# EUROPEAN PATENT SPECIFICATION

(11) **EP 1 018 420 B1**
(45) Date of publication and mention of the grant of the patent: **21.05.2003**
(21) Application number: 00300014.8
(22) Date of filing: 05.01.2000
(51) Int. Cl.: B29C 45/26, B65D 41/04

(54) **Plastic screw cap, injection mold and method for manufacturing the same**
Schraubverschlusskappe aus Kunststoff, Gussform und Verfahren zu ihrer Herstellung
Bouchon à vis en matière plastique, moule d'injection et procédé pour sa fabrication

(30) Priority: 08.01.1999 GB 9900257
(43) Date of publication of application: 12.07.2000
(73) Proprietor: DRAGON PLASTICS LIMITED, Pontypridd, Mid Glamorgan CG37 5TF (GB)
(72) Inventor: Walding, Dean, Treharris, Mid-Glamorgan CF46 5HQ (GB)
(74) Representative: Chettle, Adrian John

(56) References cited:
- EP-A- 0 345 173
- GB-A- 727 529
- US-A- 4 090 631
- US-A- 4 784 281

## Description

This invention relates to a cap, particularly a screw cap for a bottle.

Screw caps of bottles and the like are usually injection moulded in plastic. The internal screw thread requires that the fresh moulding to be unscrewed from the mould after it has cooled sufficiently. As a result injection moulds for screw caps are somewhat complex, having relatively rotatable parts as well as relatively axially movable parts.

In practice the inner mould part defining the screw thread is usually unscrewed from a relatively stationary moulding, a tubular mould part surrounding the inner mould part engaging the skirt of the cap to prevent it rotating with the inner mould part. An outer mould part, which defines the external surface of the cap is withdrawn axially in the opposite direction to the inner mould part.

This kind of mould construction is rather common, but has several disadvantages. Firstly, the unscrewing of the inner mould part whilst the mould is hot may result in a low pressure area under the top of the cap, which may cause the top to dish slightly. This problem is most noticeable in flat top caps, and may be remedied by choice of a better grade of plastic, or allowing the freshly moulded cap to cool so as to be better resistant to deformation. Dishing is not readily apparent in caps which have a raised surface design, or are not flat-topped.

A second disadvantage concerns the inevitable dogs which are moulded at the skirt edge so as to engage in corresponding dogs of the tubular mould part. These dogs are usually hidden within the cap, but nevertheless result in a skirt edge which is not entirely smooth. This disadvantage is not usually a problem because the lower skirt edge seldom has any function. Furthermore deformation of the outer skirt edge may be disguised, for example by a raised lip.

These problems must however be overcome if a high precision cap is to be moulded at rapid rates using relatively inexpensive material. Such a cap may be required for aesthetic or functional purposes, and have a very good degree of flatness and smoothness.

A high precision cap not exhibiting the disadvantages mentioned above is required in the manufacture of a metal sheathed cap, in which the plastic cap is inserted in a very thin cup-like metal sheath, the edge of which is rolled over to the interior of the plastic cap. This kind of composite cap requires a very flat top to the plastic cap in order to support the sheath, and prevent unsightly dishing. Furthermore the lower edge of the skirt must be perfectly circular and smooth in order for the sheath edge to be rolled over without unsightly wrinkles or distortion. Since such sheaths are usually required to be reflective, any distortion of the top or side would be immediately apparent under artificial light, and detract from the product to which the cap is fitted. Typically such composite caps would be for high value alcohol products, perfumes and the like where appearance is a very important factor in consumer choice.

US-A-4784281 discloses an injection mould for a screw cap corresponding to the preamble to claim 1. The cap is ejected from the mould by means of a sleeve in contact with the lower edge of the cap skirt.

US-A-4090631 discloses an injection mould for a screw cap, the skirt of which is caused to swell for ejection by a sleeve in contact with the lower edge thereof.

According to a first aspect of the invention there is provided an injection moulded plastics screw cap having a top and a skirt, a form-locking surface being moulded on the inner side of the top for non-rotational engagement of a moulding tool, characterized in that the external surface of said skirt is smooth, cylindrical and curved in a single plane only, and said skirt has a circumferential lower edge of reduced wall thickness on the inner side thereof, to define a relatively thin uniform wall, said cap further including an external metal sheath, the rim of said sheath being rolled over said edge against the inner side of said wall.

As will be understood, such a form locking surface is formed by the moulding tool as the cap is moulded, because the tool defines the mould cavity. The form locking surface may be constituted by recessed and/or raised portions, as appropriate to the cap design.

A cap of this kind has a number of advantages. Firstly, an inner part of the mould tool can retain the cap against relative rotation as a tubular thread-defining mould part is unscrewed after moulding. Accordingly dogs on the mould skirt are not necessary, and the skirt edge can thus be moulded to precisely the desired form.

Secondly, since the inner part of mould tool remains in contact with the top of the cap as the tubular mould part is unscrewed, dishing is prevented.

Thirdly, better cooling of the top of the cap through contact with the mould is ensured, and thus the risk of distortion is reduced whilst avoiding a lengthening of the mould cycle time. The flatness of the top of the cap can thus be improved. Such cooling is typically more uniform.

Fourthly, the form locking surface itself stiffens the top of the cap as compared with a plain top of uniform thickness.

Fifthly, the form locking surface is adapted to frictionally engage a liner of the cap, and prevent relative rotation thereof during use, due for example to the liner sticking to the mouth of a bottle. The form-locking surface thus ensures that the liner is removed with the cap rather than remaining on the bottle mouth.

Sixthly, the form locking surface provides recesses which are adapted to receive adhesive for the purpose of fixing a liner in the cap. Such adhesive may be necessary in cases where the contents of the bottle are rather sticky. The recesses permit a slight excess of adhesive to be accommodated, thus ensuring that the liner is always perpendicular to the rotational axis of the cap.

Other advantages come about because of better cooling of the freshly moulded cap. In particular the dimensional accuracy of the cap is better for a given mould cycle time; for example the external corner where the top joins the skirt can be much sharper, and thus is particular beneficial in the manufacture of composite caps having an outer metal sheath. Another advantage is that the form locking surface is masked when a liner is fitted, and thus the appearance of the cap is improved as compared with caps having the prior dogs on the skirt.

In a preferred embodiment the form-locking surface comprises a plurality of radial arms. However any kind of pattern is suitable provided the freshly moulded cap cannot rotate relative to the unscrewing mould part during opening of the mould tool.

Where the cap is inserted in a metal sheath, external axial grooves may be formed to allow the escape of air as the cap is pushed piston-like into the sheath.

An injection mould tool for a plastic screw cap, said cap being adapted to receive an external metal sheath having a rim rolled over the lower edge of the skirt thereof against the inner side, and the tool comprising:
an outer mould part for defining the outside of a cap to be moulded in the tool,
a tubular mould part for defining the internal thread of said cap, and a circumferential lower edge of reduced wall thickness on the inside of said cap, said lower edge defining a relatively thin uniform wall to receive the rolled edge of a sheath;
and an inner mould part for defining substantially all of the inner side of the top of said cap,
the outer and inner mould parts being relatively axially movable, the tubular mould part being relatively rotatable and relatively axially movable with respect to the inner mould part, and ejection means in the inner mould part to eject the cap from mould.

A method of injection moulding a screw cap is disclosed using a mould tool as previously defined, the method comprising the steps of
a) closing the mould tool;
b) injecting molten plastic into the cavity of the mould tool;
c) allowing the molten plastic to solidify;
d) opening the mould tool and unscrewing the tubular mould part whilst maintaining the inner mould part against the freshly moulded screw cap, and
e) ejecting the freshly moulded cap, by ejection means of the inner mould part.
Other features of the invention will be apparent from the following description of a preferred example shown by way of example only in the accompanying drawings in which:
Fig. 1 is an axial section through a cap according to the invention;
Fig. 2 is a simplified underside view of the cap of Fig. 1;
Fig. 3 is a view similar to Fig. 1 and showing a composite cap; and
Fig. 4 is a somewhat schematic drawing of a mould tool for moulding the cap of Fig. 1.

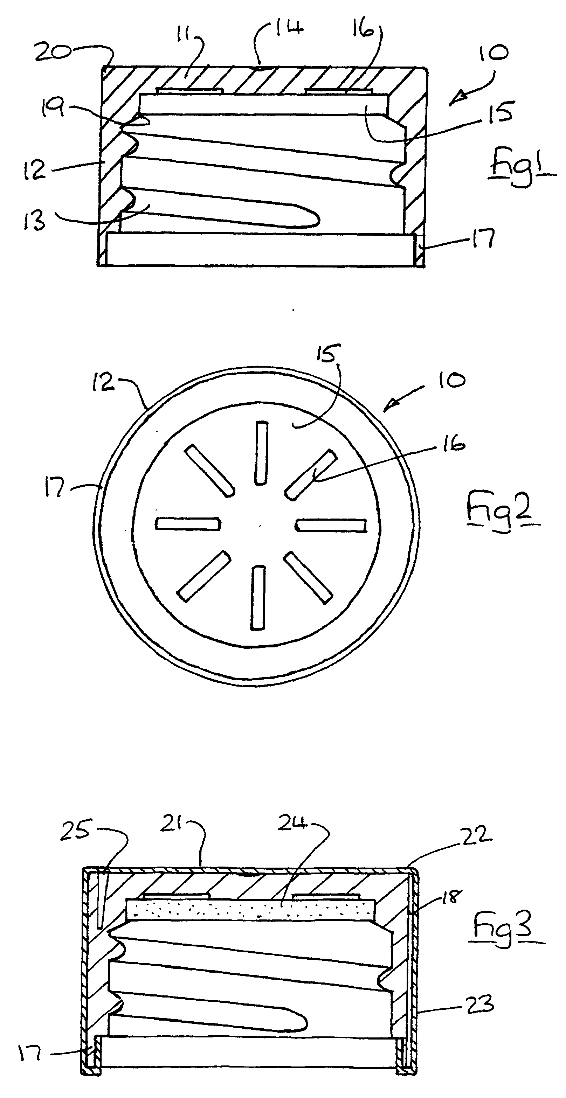

The cap illustrated in the accompanying drawings is diagrammatic, and certain features have been enlarged or omitted in order to show the invention more clearly. In particular Fig. 2 does not illustrate the internal thread form.

Figs. 1 and 2 show an injection moulded cap 10 of plastics material, such as polyethylene. The cap consists of the usual top 11, skirt 12, internal thread 13, and gate well 14. A circular recess 15 is adapted to receive a liner, and a circular array of radial recesses 16 are formed at the base of the recess 15.

The recesses 16 are defined by an inner mould tool part whereas the thread 13 is defined by a tubular mould tool part surrounding the inner mould tool part. In use the tubular mould tool part is rotated and moved axially relative to the inner mould tool part, in order to release a freshly moulded cap from the mould tool cavity, and thus allow the cap to be ejected from the mould tool. The liner recess 15 is optional.

The cap 10 has a smooth plain flat top, and a smooth plain outer skirt surface which is perfectly cylindrical. That is to say that the external surface of the skirt is very regular and curved in a single plane only. The corner between the top and the skirt is substantially a right angle.

Fig. 3 illustrates a composite cap utilising the injection moulded cap of Fig. 1 and a cup-like metal sheath 21. The sheath is drawn from sheet material, and prior to assembly consists solely of an end wall 22 and a circular skirt 23. The sheath is very thin (about 0.3 mm thick) and constitutes a decorative outer surface for the composite cap, the inner cap 10 giving the necessary strength. The sheath may carry external printed decoration and/or product information.

As illustrated the inner cap is relieved at the lower edge of the skirt on the inner side to give a very thin uniform wall 17. This thin wall is possible because, as already explained, the skirt is not required to support a fresh moulded cap against relative rotation of a conventional mould tool. The external surface of the thin wall 17 merges into the main wall of the skirt, the transition being unnoticeable to the eye.

In use the moulded cap 10 is inserted piston-like into the metal sheath, and the rim of the sheath is rolled over against the thin wall 17, thus giving a neat appearance and adding strength to the lower edge of the cap. Fig. 3 illustrates a typical narrow groove 18 of the cap 10 which allows escape of air as the cap is inserted into the sheath 21. Several such grooves may be provided for this purpose and are preferably axial so as to allow easy separation of the mould tool. A typical groove may be 0.3 mm deep and 1.2 mm wide, and four such grooves may be provided, equi-spaced around the periphery of the cap.

As illustrated the single sheath is contained entirely within the recessed portion defined by the thin wall (17). The sheath rim may not fill the recessed portion entirely, but may be bent over the skirt edge just sufficiently to be secured, typically 2-3 times the thickness of the thin wall 17 or 4-6 times the material thickness of the sheath itself.

Fig. 3 also illustrates the optional liner 24 which may be retained by adhesive, or by an inwardly directed lip 19 moulded at the mouth of the recess 15, or by both.

As already mentioned, the recesses 16 can accommodate excess liner adhesive, and may also be designed to bite into the liner so as to ensure rotation of the liner with the cap in use.

Fig. 3 clearly shows the very close conformity of the moulded cap 10 and sheath 21; this ensures that no air pockets are formed, and that there is no risk of distortion of the cap due to cavities formed between the cap and sheath. Dishing of the top 11 is avoided, and thus dishing of the top of the sheath is not possible.

The very thin wall 17 ensures that the sheath skirt can be relatively easily folded into the mouth of the cap 10, and thus unsightly distortion of the sheath skirt can be avoided. It has been found in practice that the sheath skirt can remain perpendicular to the end wall, resulting in a functional strong cap with excellent visual appearance, even when unprinted. In this respect the sharp comer at the upper edge of the cap 10 is especially important since it provides close support for the sheath in an area vulnerable to contact damage.

The composite cap may include a circular array of external recesses 25, one of which is illustrated in Fig. 3 in order to avoid a relatively thick moulded section. This structure reduces differential cooling and hence internal stresses. Each recess 25 extends circumferentially for about 25-30°, but is narrow enough at the mouth to avoid inward distortion of the sheath by contact with another object. Other edge recesses, such as the gate well 14 and grooves 18 are similarly narrow.

As illustrated the recesses 25 are arcuate and substantially triangular in the axial direction, the inner tip of each recess being approximately in the middle of the skirt wall. In the case where recesses 25 and grooves 18 are provided, these recesses and grooves are not coincident, but preferably alternated around the periphery. There may be less grooves than recesses, for example four equispaced grooves 18 and twelve equispaced recesses 25. The recesses 18 may extend into the threaded region of the skirt, typically to 50-90% of the axial depth thereof, and have an included angle of greater than 5°. Alternatively the recess 25 may be circular, extending fully around the cap, but preferably still of triangular cross-section.

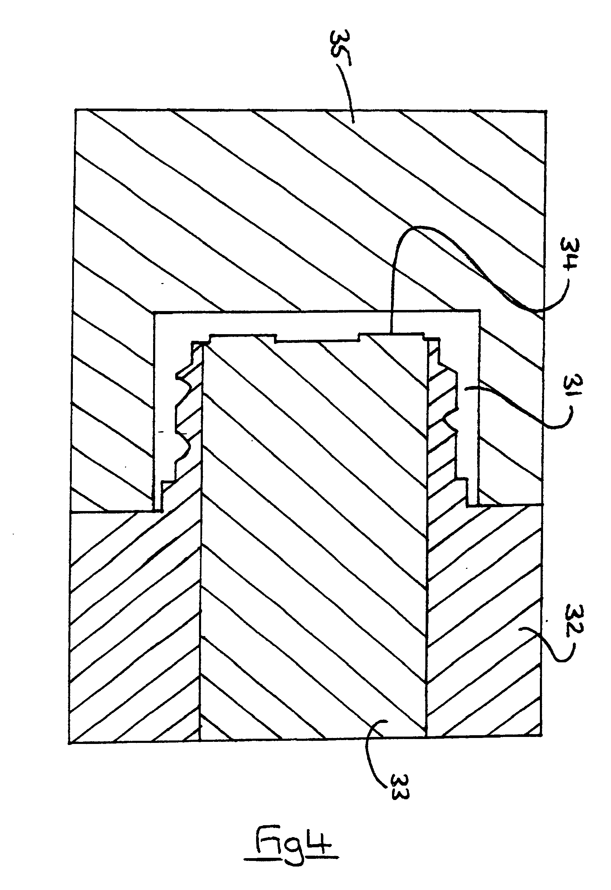

Fig. 4 illustrates a schematic mould tool for the cap of the invention, and defining a mould cavity 31. The usual injection and air ports are omitted. The mould tool is illustrated in the closed condition. To open the mould, the outer mould part 35 is withdrawn axially to expose the freshly moulded cap. The tubular mould part 32 is then rotated relative to the inner mould part 33 in order to allow the cap to unscrew from the tubular mould part. Finally the cap is ejected from the inner mould part 33 by any suitable method, e.g. by conventional ejector pins (not shown) in the inner mould part 33. During unscrewing of mould part 32, the inner mould part 33 is maintained in contact with the underside of the top of the cap, and engaged therewith by radial projections 34 (corresponding to the recesses 16); thus relative rotation of the cap with tubular mould part 32 is prevented.

## Claims

1. An injection moulded plastics screw cap (10) having a top (11) and a skirt (12), a form-locking surface (16) being moulded on the inner side of the top (11) for non-rotational engagement of a moulding tool, **characterized in that** the external surface of said skirt is smooth, cylindrical and curved in a single plane only, and said skirt (12) has a circumferential lower edge (17) of reduced wall thickness on the inner side thereof, to define a relatively thin uniform wall, said cap further including an external metal sheath (21), the rim of said sheath (21) being rolled over said edge (17) against the inner side of said wall.

2. A cap according to claim 1 wherein said form locking surface (16) extends radially of the rotational axis of said cap.

3. A cap according to claim 2 wherein said form-locking surface comprises a plurality of radial arms (16).

4. A cap according to any preceding claim and further including an external axial groove (18) on the skirt thereof.

5. An injection mould tool for a plastic screw cap, said cap being adapted to receive an external metal sheath having a rim rolled over the lower edge of the skirt thereof against the inner side, and the tool comprising:
an outer mould part (35) for defining the outside of a cap to be moulded in the tool,
a tubular mould part (32) for defining the internal thread of said cap, and a circumferential lower edge of reduced wall thickness on the inside of said cap, said lower edge defining a relatively thin uniform wall to receive the rolled edge of a sheath;
and an inner mould part (33) for defining substantially all of the inner side of the top of said cap,
the outer and inner mould parts (35,33) being relatively axially movable, the tubular mould part (32) being relatively rotatable and relatively axially movable with respect to the inner mould part (33), and ejection means in the inner mould part (33) to eject the cap from mould.

6. A method of injection moulding a screw cap using a mould tool as defined in claim 5, the method comprising the steps of
a) closing the mould tool;
b) injecting molten plastic into the cavity of the mould tool;
c) allowing the molten plastic to solidify;
d) opening the mould tool and unscrewing the tubular mould part (32) whilst maintaining the inner mould part (33) against the freshly moulded screw cap; and
e) ejecting the freshly moulded cap by ejection means of the inner mould part (33).

## Patentansprüche

1. Spritzguß-Kunststoff-Schraubkappe (10) mit einem oberen Abschnitt (11) und einer Schürze (12), wobei an der Innenseite von dem oberen Abschnitt (11) eine Form-Haltefläche (16) ausgebildet ist, um mit einem Gußform-Werkzeug unverdrehbar einzugreifen, **dadurch gekennzeichnet, dass** die Außenfläche der Schürze glatt, zylindrisch und lediglich in einer Ebene gekrümmt ist, die Schürze (12) an ihrer Innenseite eine untere Umfangskante (17) mit verminderter Wanddicke hat, um eine relativ dünne gleichmäßige Wand zu bilden, die Kappe außerdem eine äußere Metall-Ummantelung (21) aufweist, und der Rand dieser Ummantelung (21) um die Kante (17) herum gegen die Innenseite der Wand gewalzt ist.

2. Kappe nach Anspruch 1, bei der sich die Form-Haltefläche (16) in radialer Richtung von der Rotationsachse der Kappe erstreckt.

3. Kappe nach Anspruch 2, bei der die Form-Haltefläche eine Vielzahl von radial verlaufenden Schenkeln (16) aufweist.

4. Kappe nach einem der vorhergehenden Ansprüche, die außerdem an ihrer Schürze eine äußere axial verlaufende Nut (18) aufweist.

5. Spritzguss-Formwerkzeug für eine Kunststoff-Schraubkappe, wobei die Kappe dazu ausgestaltet ist, eine äußere Metall-Ummantelung aufzunehmen, die einen Rand hat, der um die untere Kante von deren Schürze herum gegen die Innenseite gewalzt ist, und wobei das Werkzeug aufweist:
ein äußeres Form-Teil (35) zum Formen der Außenseite einer in dem Werkzeug zu formenden Kappe,
ein rohrförmiges Form-Teil (32) zum Formen des Innengewindes der Kappe und einer unteren Umfangskante mit verminderter Wanddicke an der Innenseite der Kappe, wobei die untere Kante eine relativ dünne gleichmäßige Wand bildet, um die gewalzte Kante der Ummantelung aufzunehmen,
und ein inneres Form-Teil (33) zum Formen nahezu der gesamten inneren Seite des oberen Abschnitts der Kappe,
wobei das äußere und das innere Form-Teil (35, 33) relativ zueinander axial verlagerbar sind, wobei das rohrförmige Form-Teil (32) bezüglich des inneren Form-Teils (33) relativ verdrehbar und relativ axial verlagerbar ist, und Auswurf-Einrichtungen in dem inneren Form-Teil (33), um die Kappe aus der Form auszuwerfen.

6. Verfahren zum Spitzgiessen einer Schraubkappe unter Verwendung eines Form-Werkzeugs nach Anspruch 5, wobei das Verfahren die Schritte aufweist:
a) Schliessen des Form-Werkzeugs,
b) Spritzen von geschmolzenem Kunststoff in den Hohlraum des Form-Werkzeugs,
c) Ermöglichen, dass sich der geschmolzene Kunststoff verfestigt,
d) Öffnen des Form-Werkzeugs und Herausschrauben des rohrförmigen Form-Teils (32), während das innere Form-Teil (30) weiterhin gegen die frisch geformte Schraubkappe gedrückt bleibt, und
e) Auswerfen der frisch geformten Kappe durch die Auswurfeinrichtungen des inneren Form-Teils (33).

## Revendications

1. Bouchon à vis en matière plastique moulé par injection (10) ayant une partie supérieure (11) et une jupe (12), une surface à configuration de verrouillage (16) étant moulée du côté intérieur de la partie supérieure (11) pour engagement non tournant d'un outil de moulage, **caractérisé en ce que** la surface extérieure de ladite jupe est lisse, cylindrique et courbe dans un seul plan, et ladite jupe (12) présente un bord inférieur circonférentiel (17) d'épaisseur de paroi réduite sur son côté intérieur, de façon à définir une paroi uniforme relativement mince, ledit bouchon comprenant en outre une gaine en métal extérieure (21), le rebord de ladite gaine (21) étant rabattu autour dudit bord (17) contre le côté intérieur de ladite paroi.

2. Bouchon selon la revendication 1, dans lequel ladite surface à configuration de verrouillage (16) s'étend radialement par rapport à l'axe de rotation dudit bouchon.

3. Bouchon selon la revendication 2, dans lequel ladite surface à configuration de verrouillage comprend une pluralité de bras radiaux (16).

4. Bouchon selon une quelconque des revendications précédentes, comprenant en outre une rainure extérieure axiale (18) dans sa jupe.

5. Outil de moulage par injection pour bouchon à vis en matière plastique, ledit bouchon étant prévu pour recevoir une gaine en métal extérieure ayant un rebord rabattu autour du bord inférieur de sa jupe, contre le côté intérieur, et l'outil comprenant :
une partie extérieure de moule (35), pour définir l'extérieur d'un bouchon à mouler dans l'outil,
une partie tubulaire de moule (32), pour définir le filetage intérieur dudit bouchon et un bord inférieur circonférentiel d'épaisseur de paroi réduite du côté intérieur dudit bouchon, ledit bord inférieur définissant une paroi uniforme relativement mince pour recevoir le bord rabattu d'une gaine ; et
une partie intérieure de moule (33), pour définir sensiblement la totalité du côté intérieur de la paroi supérieure dudit bouchon,
les parties de moule extérieure et intérieure (35, 33) étant déplaçables axialement l'une par rapport à l'autre, la partie tubulaire de moule (32) pouvant tourner relativement et se déplacer relativement axialement par rapport à la partie intérieure de moule (33), et des moyens d'éjection étant prévus dans la partie intérieure de moule (33) pour éjecter le bouchon du moule.

6. Procédé de moulage par injection d'un bouchon à vis au moyen d'un outil de moulage selon la revendication 5, le procédé comprenant les étapes suivantes :
(a) fermer l'outil de moule ;
(b) injecter de la matière plastique fondue dans la cavité de l'outil de moule ;
(c) Laisser la matière plastique fondue se solidifier ;
(d) ouvrir l'outil de moule et dévisser la partie tubulaire de moule (32) tandis que la partie intérieure de moule (33) est maintenue contre le bouchon à vis qui vient d'être moulé ; et
(e) éjecter le bouchon qui vient d'être moulé, par des moyens d'éjection prévus dans la partie intérieure du moule (33).
